# EUROPEAN PATENT SPECIFICATION

(11) **EP 1 870 347 B1**
(45) Date of publication and mention of the grant of the patent: **15.06.2011**
(21) Application number: 07380135.9
(22) Date of filing: 11.05.2007
(51) Int. Cl.: B65D 51/00, B65D 51/18, A61J 1/00

(54) **Stopper for flasks of sterile products and use of said stopper in sterile measured filling**
Stopfen für Flaschen mit sterilen Produkten und Verwendung dieses Stopfens für eine abgemessene sterile Füllung
Butoir pour flacons de produits stériles et utilisation de ce butoir pour un remplissage mesuré stérile

(30) Priority: 19.06.2006 ES 200601691
(43) Date of publication of application: 26.12.2007
(73) Proprietor: Grifols, S.A., 08022 Barcelona (ES)
(72) Inventor: López Alvarez, Diego, 08530 La Garriga (Barcelona) (ES); Roura Adell, Sergi, 08358 Arenys de Munt (Barcelona) (ES); Sánchez Sabaté, José Ramón, 08150 Parets del Vallés (ES)
(74) Representative: Durán Moya, Luis-Alfonso

(56) References cited:
- EP-A1- 0 837 008
- ES-A1- 2 232 269
- ES-A6- 2 016 490
- US-A- 5 314 084
- US-A- 5 718 348
- US-A- 5 901 866

## Description

The present invention relates to a stopper for flasks according to the preamble of claim 1, especially for flasks of sterile products, and to the use of same in a method of sterile measured filling of flasks.

More particularly, the stopper of the present invention is especially suitable as a stopper for vials the sterile contents of which are sealed and are to be administered by injection.

However, specific embodiments of the present invention are not necessarily confined to the applications cited.

The document US5718348 discloses a device according to the preamble of claim 1 meant to perform a process similar to the one described above by implementing a sealing plug and a protective cover used in conjunction with a flask with two protuberances. A first entity inserts this sealing plug from a flared part of the protective cover until it reaches a rib, then, a second entity locates the protective cover with the sealing plug over the flask making a tab engage the first protuberance located on the mouth of the flask. Then, after the flask is lyophilized, the device is pressed downwardly to make the plug reach a recess and get in a sealed position with respect to the protective cover and finally the flask is filled and the device is put over the flask and press downwardly until the tab engages the second protuberance located on the neck of the flask.

Furthermore the lyophilization process described on document US5718348 is made with the plug covering the mouth of the flask and there is no guarantee that the plug does not cover completely the mouth of the flask, being not ensured the entrance of cleaning means into the flask.

The documents ES2016490 and ES2232269 disclose a method of sterile measured filling of flasks, preferably of vials, which comprises the steps of placing a stopper on a flask in a pre-stoppered position, placing a protector thereon, sterilising the flask in an autoclave with the stopper and the protector in a pre-closed position, cooling the flask-stopper-protector assembly, removal of the protector and the stopper, measured filling of the contents of the container into the latter, and closing the flask with the stopper. During the process, the protector and the stopper exert a labyrinth closure action which permits the sterilisation of the interior of the container in the autoclave, but prevents the entry of particles which might be present in the non-sterile air.

After the measured filling, the stopper is inserted fully into the mouth of the container. Since the stopper includes a metal capsule, the final sealing of the container is obtained by deformation of said capsule (encapsulation or beading of the capsule). In accordance sterility of the whole owing to the labyrinth closure effect which is established between the skirts or walls of the protector and the wall of the flask or vial.

It is an aim of the present invention to disclose means which permit the simplification, reduction of costs and increase in safety in the aforesaid method.

For this purpose, the present invention discloses a stopper for a flask, especially a flask for pharmaceutical products, characterized in that it comprises, in combination,
- a plug with resilient means for adjustment to the inner face of the neck of a flask and an upper surface for stoppering the aperture or mouth of the flash;
- means for defining a position for pre-stoppering of the flask, before the stoppered position, in which the resilient adjustment means are not fully inside the neck of the bottle;
- a protector joined to the plug which comprises a lateral wall, said lateral wall having on its inner face a projection for engagement with an annular protuberance forming the mouth of the container in the aforesaid stoppered position;
the aforesaid protector having an extension starting from the aforesaid projection in order to form, together with the transition zone between the neck and the main body of the flask, in the pre-stoppered position, a labyrinth closure allowing steam to enter the flask but keeping the particles in suspension in the air surrounding the flask from entering.

Preferably, in order to perform the required functions, the length of the extension of the lateral wall of the protector, from the upper point of the projection to its free end, will be equal to or greater than the travel of the stopper between the stoppered position and the pre-stoppered position.

The stopper of the present invention has the advantage of providing a labyrinth type closure against the particles in suspension in the air during the aforesaid processes of sterile measured filling, both in the stoppered position and in the pre-stoppered position. However, given their different physical states, the labyrinth closure does not prevent the sterilising steam in the autoclave from penetrating, thereby making it possible to sterilise the flask in the pre-stoppered position, minimising the time for which the inside of the flask is exposed to the ambient air during the measured filling process. On the other hand, the stopper of the present invention, by having the plug and the protector already joined to each other, provides the system with the following savings:
- of logistical type: it is only necessary to provide and handle one part where two were handled before.
- of economic type, in addition, since owing to the protuberance of the inside of the lateral wall of the protector it is not necessary to carry out the operation of closure by deformation of the metal capsule (beading).

This brings with it a significant saving of time, simplification of the equipment, and the reduction of overall costs.

Another additional advantage of the present invention lies in the fact that the conventional beading of the metal capsules generates particles which require the separation of the processes of measured filling and beading, an action which is unnecessary with the present invention.

The extension of the lateral wall may be defined as an extension of the projection of the lateral wall, as a straight extension of the lateral wall, as a conical extension below the projection of the lateral wall, or as a combination of a conical extension with a cylindrical end.

The stopper of the present invention in addition makes it possible to arrange an upper pull-off lid on the stopper. In this case, the protector may have an upper lid which gives access, via a window, to the plug made of rubbery material (rubber). This preferred embodiment will be directed especially to vials for sterile pharmaceutical products for intravenous administration.

As a consequence of all the above, the present invention also comprises the use of the stopper of the present invention in a process of sterile measured filling of flasks for pharmaceutical products. Preferably this process comprises, at least, the following steps:
- placing the stopper on the flask, in a pre-stoppered position,
- sterilisation in an autoclave, preferably with vacuum cycles,
- preferably, a cooling step,
- extraction of the stopper,
- measured filling of the contents of the flask,
- closure of the flask, placing the stopper in the stoppered position.

This method will be especially useful in the case where the flask is a vial for pharmaceutical products for intravenous administration.

For greater understanding of the invention, drawings of some embodiments of the present invention are appended by way of non-limiting example.
Figure 1 shows a front view of a flask assembly for a pharmaceutical product, stoppered by means of a stopper according to the present invention.
Figure 2 shows a cross-section through the assembly of Figure 1.
Figure 3 shows a detail of the stopper of Figure 2.
Figures 4, 5 and 6 show three alternative embodiments of the stopper according to the present invention.
Figure 7 shows diagrammatically a process of sterilisation and measured filling in which a stopper according to the present invention is used.

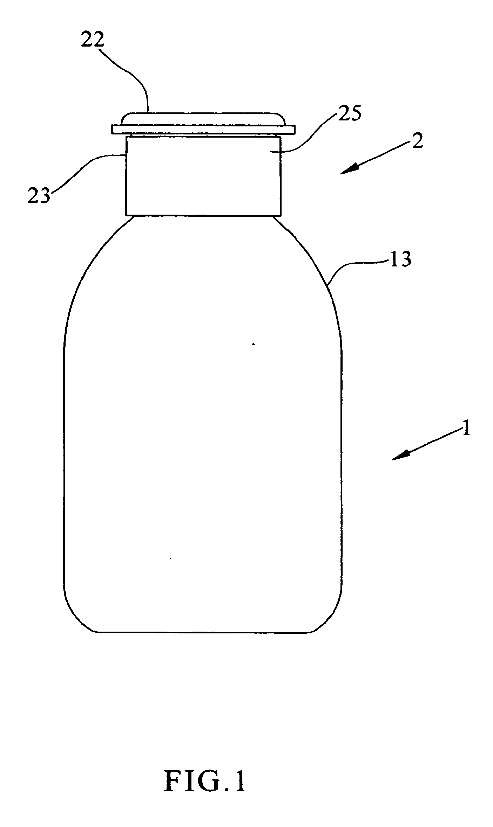

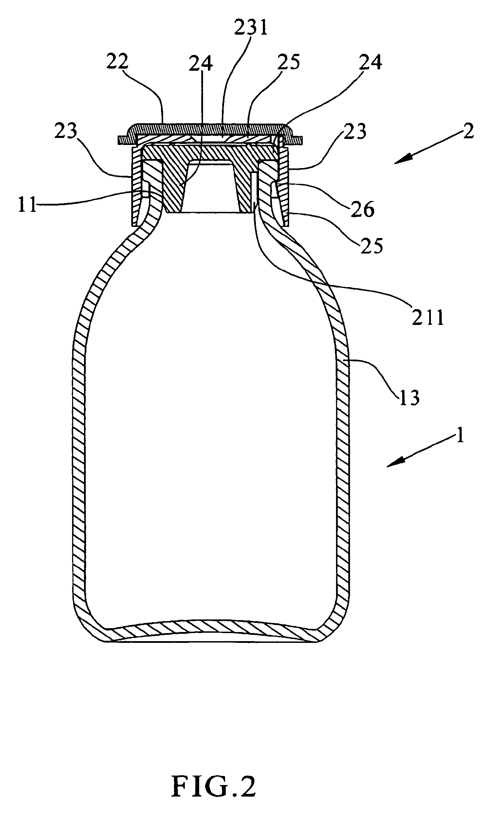

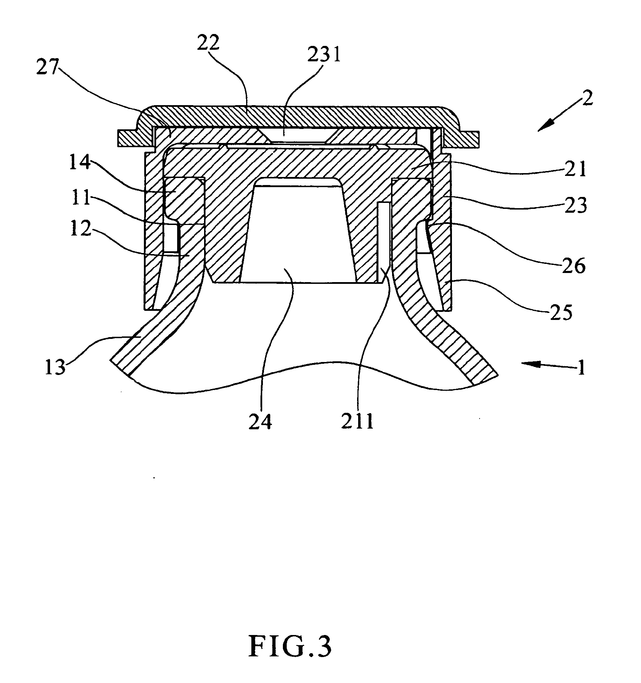

In Figures 1 to 3 an exemplary embodiment of the stopper 2 of the present invention can be seen in the stoppered position, closing a vial 1 for medicinal products.

As can be seen from the outside, the stopper 2 has an upper lid 22 which can be removed by being pulled off, and a lateral wall 23 which has an extension 25, which in the examples shown extends almost to touch the shoulder 13 (transition zone between the neck 12 and the main body of the vial 1), in such a way that the small gap between the shoulder 13 and the extension 25 forms a labyrinth closure for the ambient air, since it is complicated for any possible particles present in the ambient air to pass through the existing gap in a direction opposed to gravity.

Internally, the stopper 2 shown in the examples is composed of a plug 24, made of an elastomer, such as rubber. The plug 24 may have a special geometry which allows the stopper to rest in the mouth of the container (pre-stoppering) owing to a stepped contour (not shown). In addition, the plug 24 may have grooves 211 to permit, in the pre-stoppered position, the passage and emergence of the steam which will sterilise the container in the autoclave. The plug 24 also comprises an upper wall 21 for stoppering the bottle. This wall 21, made of rubbery material, makes it possible to be traversed by the needle of a syringe for the extraction of the pharmaceutical product from the interior without the need to open the stopper, which would expose the contents to the atmosphere.

The stopper shown also has a protector joined to the plug by insertion or assembly, or by any other means, of the plug inside the protector in a previous process not described herein. The protector comprises the aforesaid lateral wall 23, which will preferably be made of synthetic material, such as a plastics material, and the removable lid 22. Said lateral wall has on its inner wall a projection 26 for engagement with the annular protuberance 14 of the neck of the vial 1 in the mouth region. Said projection may be arranged along the entire perimeter of the inner face of the lateral wall 23 or at a specific number of points. Starting from the aforesaid projection 26, the lateral wall 23 has an extension 25 which, in the example of Figures 1 to 3, is in the shape of an extension of the projection and the free end of which extends so that it almost touches the shoulder 13 of the vial 1. In this way, a labyrinth closure is established against the contaminating particles which may be suspended in the ambient air.

In use, with the stopper in the closed position shown in Figures 1 to 6, it is possible to pull off the upper lid 22, which in this way acts as an upper pull-off enclosure. Beneath said enclosure 22 there is another upper lid 27 which has a window 231 which gives access to the upper wall 21 of the rubber plug 24, allowing it to be punctured by the needle of a syringe.

Similarly, in the example shown, the lower end of the plug 24 has an inclined/stepped wall which makes it possible to define a pre-stoppered position of the flask, in which the stopper rests, for example, on the inner edge of the mouth of the container 14 and the inner surface of the neck 11 of the container. In said position, it is preferable for the extension of the lateral wall 25 to have a length, from the upper point of the projection 26 to its free end, equal to or greater than the travel of the stopper 2 between the pre-stoppered position and the stoppered position. In this way, in the pre-stoppered position, the extension 25 and the annular protuberance of the mouth 14 of the container are close to each other, almost touching each other, so that in the pre-stoppered position the extension 25 of the lateral wall 23 also forms with the flask 1 a labyrinth closure with respect to the particles in suspension in the air.

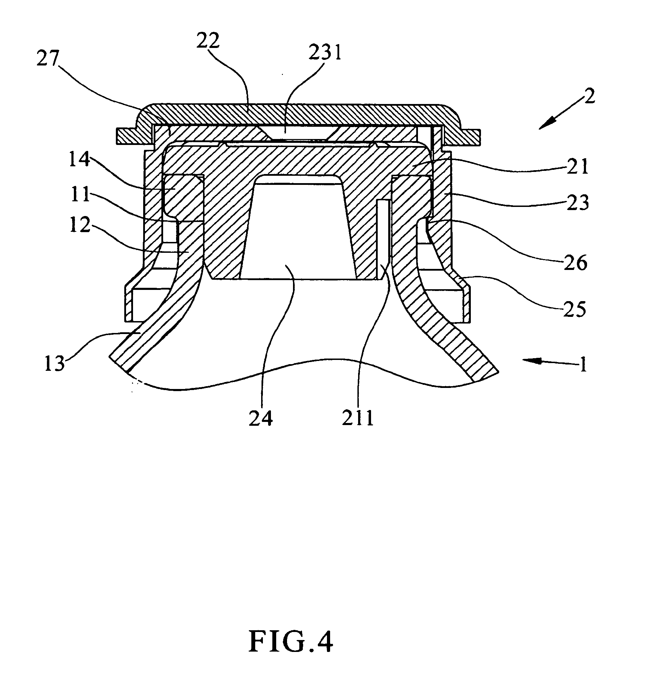

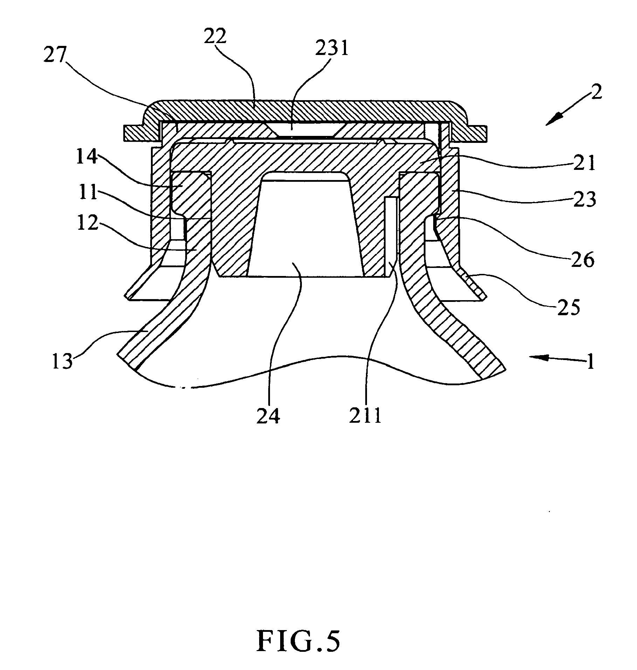

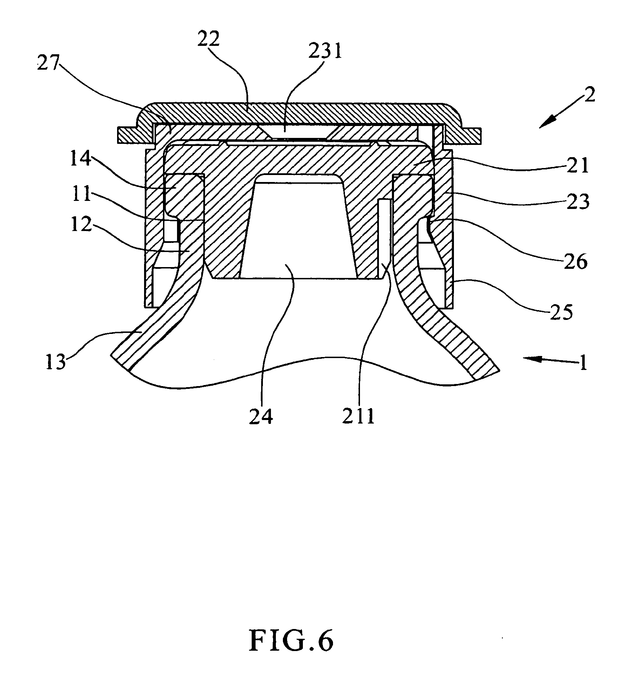

Naturally, the extension 25 of the lateral wall of the stopper 2 may be of various shapes. In Figure 4, the extension 25 has a conical section with a cylindrical free end zone. In Figure 5, the extension 25 is conical in shape on the outside. In Figure 6, the extension 25 is cylindrical in shape on the outside and its free end is likewise cylindrical.

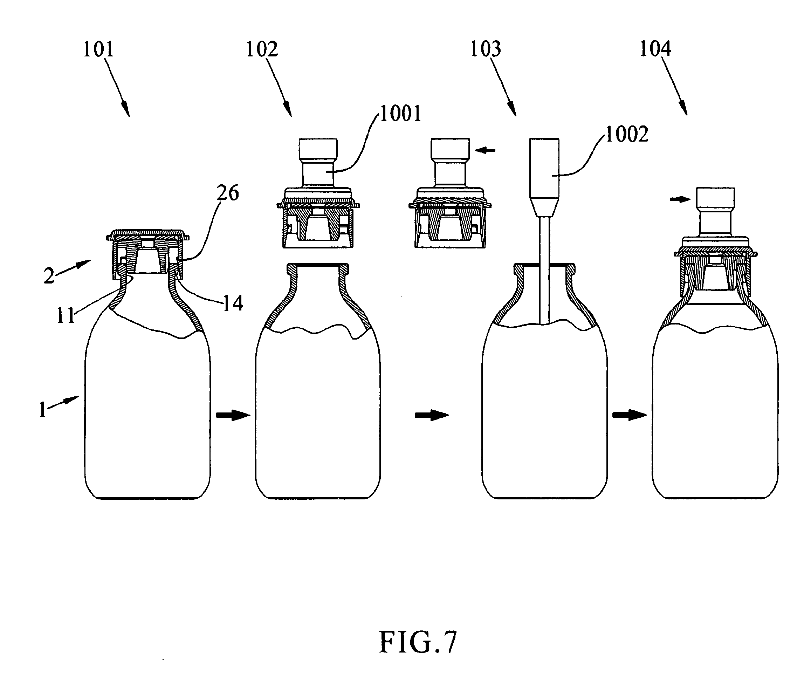

Figure 7 shows an example of the use of a stopper 2 according to the present invention in a flask for pharmaceutical products, more specifically a vial 1.

The method shown includes a first step of placing the stopper 2 on the flask 1 in a pre-stoppered position; in this position a step 101 of sterilisation in an autoclave is then carried out. After the process of sterilisation in an autoclave and, optionally, cooling, the measured filling of the pharmaceutical product into the flask takes place, for which a first sub-step 102 of removal of the stopper (plug and protector) is carried out with a single implement 1001, followed by a second sub-step of filling 103 with a specific implement 1002 and then closure 104 of the stopper 2 on the flask 1 as far as the closed position, which can be performed, if desired, by means of the same implement 1001 which was used to separate the stopper from the flask. Once the container is closed, no beading operation is required, since the sealing thereof is obtained by clipping the protector onto the container.

It is therefore clear that the significant economy which is obtained in this method by using the stopper of the present invention, in comparison with methods of known type, in which use was made of a stopper (which included a metal capsule) and a protector as two separate parts, without any join.

Although a description has been given of some examples of preferred use of the stopper described, the invention is not necessarily limited to same. In particular, although applications have been shown in which the flask has a narrowed neck, said stopper may also be applied to flasks in which the neck has the same dimensions as the main body of the flask.

Although the invention has been described with respect to preferred exemplary embodiments, these should not be regarded as limiting the invention, which will be defined by the widest interpretation of the following claims.

## Claims

1. A flask stopper **(12),** which comprises, in combination:
- a plug **(24)** with resilient means for adjustment to the inner face of the neck of a flask and an upper surface for stoppering the opening or mouth of the flask;
- means for defining a position for pre-stoppering of the flask, before the stoppered position, in which the resilient adjustment means are not fully inside the neck of the bottle; **characterized in that** it comprises
- a protector **(23,27)** joined to the plug **(24)** which comprises a lateral wall, said lateral wall having on its inner face a projection for engagement with the annular protuberance forming the mouth of the container in the aforesaid stoppered position;
the aforesaid protector having an extension starting from the aforesaid projection to form, together with the transition zone between the neck and the main body of the flask, in the pre-stoppered position, a labyrinth closure allowing steam to enter the flask but keeping the particles in suspension in the air surrounding the flask from entering.

2. A stopper **(2)** according to claim 1, **characterized in that** the length of the extension of the lateral wall of the protector, from the upper point of the projection to its free end, is equal to or greater than the travel of the stopper **(2)** between the stoppered position and the pre-stoppered position.

3. A stopper **(2)** according to claim 1 or claim 2, **characterized in that** the aforesaid extension is cylindrical in shape on the outside.

4. A stopper **(2)** according to claim 1 or claim 2, **characterized in that** the aforesaid extension is conical in shape on the outside.

5. A stopper **(2)** according to claim 1 or claim 2, **characterized in that** the aforesaid extension has a conical section with a cylindrical free end zone.

6. A stopper **(2)** according to any one of claims 1 to 5, **characterized in that** it has a pull-off upper enclosure.

7. A stopper **(2)** according to any one of claims 1 to 6, **characterized in that** the protector has an upper lid (22) with a window which gives access to the plug.

8. A stopper **(2)** according to any one of claims 1 to 7, **characterized in that** the plug **(24)** is made of rubber.

9. A stopper **(2)** according to any one of claims 1 to 8, **characterized in that** the protector is made of synthetic material.

10. A stopper **(2)** according to any one of claims 1 to 9, **characterized in that** the means for defining a pre-stoppering position comprise a stepped contour on the plug.

11. A stopper **(2)** according to any one of claims 1 to 10, **characterized in that** the plug **(24),** in the pre-stoppered position, forms a labyrinth closure together with the inner face of the annular rim of the mouth of the flask.

12. A stopper **(2)** according to any one of claims 1 to 11, **characterized in that** the plug **(24)** has a groove **(211)** which, in the pre-stoppered position, permits the passage and emergence of steam from inside the flask.

13. The use of a stopper according to any one of claims 1 to 12 in a method of sterile measured filling of flasks of pharmaceutical products.

14. The use according to claim 13, **characterized in that** the method of sterile measured filling comprises the following steps:
- placing the stopper on the flask, in the pre-stoppered position;
- sterilisation in an autoclave;
- measured filling of the pharmaceutical product into the flask;
- closing the flask with the stopper, as far as its stoppered position.

15. The use according to claim 14, in which sterilisation takes place with the stopper on the flask, in its pre-stoppered position.

16. The use according to any one of claims 13 to 15, **characterized in that** the flask is a vial for pharmaceutical products for intravenous administration.

17. The use according to any one of claims 14 to 16, **characterized in that** the method proceeds to a cooling step between the sterilisation in an autoclave and the measured filling of the product.

## Patentansprüche

1. Flaschenstöpsel (12), welcher in Kombination umfasst:
- einen Stopfen (24) mit nachgiebigen Mitteln zur Anpassung an die innere Fläche des Halses einer Flasche und einer oberen Oberfläche zum Zustöpseln der Öffnung oder Mündung der Flasche;
- Mittel zum Definieren einer Position zum Vor-Zustöpseln der Flasche vor der zugestöpselten Position, in welcher die nachgiebigen Anpassungsmittel nicht vollständig in dem Hals der Flasche sind; **dadurch gekennzeichnet, dass** er umfasst:
- eine mit dem Stopfen (24) verbundene Schutzeinrichtung (23, 27), welche eine seitliche Wand umfasst, wobei die seitliche Wand an ihrer inneren Fläche einen Vorsprung zum Zusammenwirken mit der ringförmigen Ausstülpung aufweist, welche die Mündung des Behälters bildet, in der zugestöpselten Position;
wobei die Schutzeinrichtung eine Verlängerung aufweist, welche an dem Vorsprung beginnt, um zusammen mit dem Übergangsbereich zwischen dem Hals und dem Hauptkörper der Flasche, in der vor-zugestöpselten Position, einen Labyrinthverschluss zu bilden, welcher es Dampf erlaubt, in die Flasche einzutreten, aber die Partikel in Suspension in der die Flasche umgebenden Luft davon abhält, einzutreten.

2. Stöpsel (2) nach Anspruch 1, **dadurch gekennzeichnet, dass** die Länge der Verlängerung der seitlichen Wand der Schutzeinrichtung von dem oberen Punkt des Vorsprungs bis zu ihrem freien Ende gleich oder größer als der Hubweg des Stöpsels (2) zwischen der zugestöpselten Position und der vor-zugestöpselten Position ist.

3. Stöpsel (2) nach Anspruch 1 oder Anspruch 2, **dadurch gekennzeichnet, dass** die Verlängerung außen eine zylindrische Form aufweist.

4. Stöpsel (2) nach Anspruch 1 oder Anspruch 2, **dadurch gekennzeichnet, dass** die Verlängerung außen eine konische Form aufweist.

5. Stöpsel (2) nach Anspruch 1 oder Anspruch 2, **dadurch gekennzeichnet, dass** die Verlängerung einen konischen Abschnitt mit einem zylindrischen freien Endbereich aufweist.

6. Stöpsel (2) nach einem der Ansprüche 1 bis 5, **dadurch gekennzeichnet, dass** er eine obere Abziehabdeckung aufweist.

7. Stöpsel (2) nach einem der Ansprüche 1 bis 6, **dadurch gekennzeichnet, dass** die Schutzeinrichtung einen oberen Deckel (22) mit einem Fenster aufweist, welches Zugang zu dem Stopfen gewährt.

8. Stöpsel (2) nach einem der Ansprüche 1 bis 7, **dadurch gekennzeichnet, dass** der Stopfen (24) aus Gummi besteht.

9. Stöpsel (2) nach einem der Ansprüche 1 bis 8, **dadurch gekennzeichnet, dass** die Schutzeinrichtung aus synthetischem Material besteht.

10. Stöpsel (2) nach einem der Ansprüche 1 bis 9, **dadurch gekennzeichnet, dass** die Mittel zum Definieren einer vor-zugestöpselten Position eine abgestufte Kontur auf dem Stopfen aufweisen.

11. Stöpsel (2) nach einem der Ansprüche 1 bis 10, **dadurch gekennzeichnet, dass** der Stopfen (24) in der vor-zugestöpselten Position zusammen mit der inneren Fläche des ringförmigen Randes der Mündung der Flasche einen Labyrinthverschluss bildet.

12. Stöpsel (2) nach einem der Ansprüche 1 bis 11, **dadurch gekennzeichnet, dass** der Stopfen (24) eine Nut (211) aufweist, welche in der vor-zugestöpselten Position den Durchtritt und Austritt von Dampf von innerhalb der Flasche erlaubt.

13. Verwendung eines Stöpsels nach einem der Ansprüche 1 bis 12 in einem Verfahren zum sterilen abgemessenen Befüllen von Flaschen von pharmazeutischen Produkten.

14. Verwendung nach Anspruch 13, **dadurch gekennzeichnet, dass** das Verfahren zum sterilen abgemessenen Befüllen die folgenden Schritte umfasst:
- Platzieren des Stöpsels auf der Flasche in der vor-zugestöpselten Position;
- Sterilisation in einem Autoklaven;
- abgemessenes Befüllen des pharmazeutischen Produkts in die Flasche;
- Schließen der Flasche mit dem Stöpsel bis zu seiner zugestöpselten Position.

15. Verwendung nach Anspruch 14, bei welcher Sterilisation mit dem Stöpsel auf der Flasche in seiner vor-zugestöpselten Position stattfindet.

16. Verwendung nach einem der Ansprüche 13 bis 15, **dadurch gekennzeichnet, dass** die Flasche eine Ampulle für pharmazeutische Produkte zur intravenösen Verabreichung ist.

17. Verwendung nach einem der Ansprüche 14 bis 16, **dadurch gekennzeichnet, dass** das Verfahren mit einem Kühlungsschritt zwischen der Sterilisation in einem Autoklaven und dem abgemessenen Befüllen des Produkts fortfährt.

## Revendications

1. Obturateur (12) de flacon, qui comprend, en combinaison :
un bouchon (24) avec des moyens élastiques pour l'ajustement sur la surface interne du goulot d'un flacon et une surface supérieure pour obturer l'ouverture ou embouchure du flacon ;
des moyens pour définir une position de pré-obturation du flacon, avant la position obturée, dans laquelle les moyens d'ajustement élastiques ne sont pas complètement à l'intérieur du goulot de la bouteille ; **caractérisé en ce qu'**il comprend :
un dispositif de protection (23, 27) assemblé au bouchon (24) qui comprend une paroi latérale, ladite paroi latérale ayant sur sa face interne, une saillie pour la mise en prise avec la protubérance annulaire formant l'embouchure du récipient dans la position obturée mentionnée précédemment ;
ledit dispositif de protection mentionné précédemment ayant une extension à partir de la saillie mentionnée précédemment afin de former, conjointement avec la zone de transition entre le goulot et le corps principal du flacon, dans la position pré-obturée, une fermeture à labyrinthe permettant à la vapeur d'entrer dans le flacon mais empêchant les particules en suspension dans l'air autour du flacon, d'entrer.

2. Obturateur (2) selon la revendication 1, **caractérisé en ce que** la longueur de l'extension de la paroi latérale du dispositif de protection, à partir du point supérieur de la saillie jusqu'à son extrémité libre, est égale ou supérieure au déplacement de l'obturateur (2) entre la position obturée et la position pré-obturée.

3. Obturateur (2) selon la revendication 1 ou la revendication 2, **caractérisé en ce que** l'extension mentionnée précédemment a une forme cylindrique sur l'extérieur.

4. Obturateur (2) selon la revendication 1 ou la revendication 2, **caractérisé en ce que** l'extension mentionnée précédemment a une forme conique à l'extérieur.

5. Obturateur (2) selon la revendication 1 ou la revendication 2, **caractérisé en ce que** l'extension mentionnée précédemment a une section conique avec une zone d'extrémité libre cylindrique.

6. Obturateur (2) selon l'une quelconque des revendications 1 à 5, **caractérisé en ce qu'**il a une enceinte supérieure de rupture.

7. Obturateur (2) selon l'une quelconque des revendications 1 à 6, **caractérisé en ce que** le dispositif de protection a un couvercle supérieur (22) avec une fenêtre qui donne accès au bouchon.

8. Obturateur (2) selon l'une quelconque des revendications 1 à 7, **caractérisé en ce que** le bouchon (24) est réalisé à partir de caoutchouc.

9. Obturateur (2) selon l'une quelconque des revendications 1 à 8, **caractérisé en ce que** le dispositif de protection est réalisé avec un matériau synthétique.

10. Obturateur (2) selon l'une quelconque des revendications 1 à 9, **caractérisé en ce que** les moyens pour définir une position pré-obturée comprennent un contour étagé sur le bouchon.

11. Obturateur (2) selon l'une quelconque des revendications 1 à 10, **caractérisé en ce que** le bouchon (24), dans la position pré-obturée, forme une fermeture à labyrinthe conjointement avec la face interne de la collerette annulaire de l'embouchure du flacon.

12. Obturateur (2) selon l'une quelconque des revendications 1 à 11, **caractérisé en ce que** le bouchon (24) a une rainure (211) qui, dans la position pré-obturée, permet le passage et la sortie de la vapeur de l'intérieur du flacon.

13. Utilisation d'un obturateur selon l'une quelconque des revendications 1 à 12 dans un procédé de remplissage mesuré stérile de flacons de produits pharmaceutiques.

14. Utilisation selon la revendication 13, **caractérisée en ce que** le procédé de remplissage mesuré stérile comprend les étapes suivantes consistant à :
placer l'obturateur sur le flacon, dans la position pré-obturée ;
stériliser dans un autoclave ;
déverser le produit pharmaceutique mesuré dans le flacon ;
fermer le flacon avec l'obturateur, tant qu'il est dans sa position obturée.

15. Utilisation selon la revendication 14, dans laquelle la stérilisation a lieu avec l'obturateur sur le flacon, dans sa position pré-obturée.

16. Utilisation selon l'une quelconque des revendications 13 à 15, **caractérisée en ce que** le flacon est un flacon pour les produits pharmaceutiques pour l'administration par voie intraveineuse.

17. Utilisation selon l'une quelconque des revendications 14 à 16, **caractérisée en ce que** le procédé continue par une étape de refroidissement entre la stérilisation dans une autoclave et le remplissage mesuré du produit.
